# EUROPEAN PATENT APPLICATION

(11) **EP 4 830 655 A1**
(43) Date of publication of application: **28.10.2026**
(21) Application number: 25172631.1
(22) Date of filing: 25.04.2025
(51) Int. Cl.: A61B 18/24

(54) **LASER CATHETER HAVING EXPANDING DISTAL END**

(71) Applicant: Koninklijke Philips N.V., 5656 AE Eindhoven (NL)
(72) Inventor: CONANT, Brandi, Eindhoven (NL)
(74) Representative: Philips Intellectual Property & Standards

(57) **Abstract**

A medical device includes a catheter having a retractable sheath disposed over a guidewire. The catheter is adapted to transmit laser light energy in a contacting medical procedure. The medical device further includes an expansion device adapted to expand an optical fiber bundle from a first diameter to a second diameter.

## Description

### BACKGROUND

Fatty deposits can form in arteries, and can cause occlusion and interfere or restrict blood flow through the vessel, causing burden on the cardiovascular system, and restriction of blood flow to certain body areas.

Atherectomy is a minimally invasive medical procedure used to remove plaque buildup from the inside of arteries. Atherectomy is used to treat conditions such as peripheral artery disease (PAD), coronary artery disease, and aortic aneurysm. The procedure can improve blood flow, reduce symptoms such as pain or cramping, and prevent complications such as heart attack or stroke.

In known atherectomy procedures, a catheter is inserted into the artery. The catheter is equipped with a device, such as a rotating blade or laser, that cuts or shaves away the plaque. Often, the blade or optical fiber transmitting the laser energy contact the plaque, and ablates the plaque. The removed plaque is then collected and removed from the body.

While known catheter devices have shown promise in atherectomy procedures, their use is generally limited to comparatively small areas at a time. Accordingly, the complexity and duration of a procedure needed to remove a comparatively large area of plaque or other lesion limits the attractiveness of known medical devices used for such procedures.

What is needed, therefore, is a medical device that enables atherectomy procedures that overcomes at least the noted drawbacks of the known approaches described above.

### SUMMARY

In accordance with a representative embodiment, a medical device is disclosed. The medical device comprises a catheter comprising: a retractable sheath disposed over a guidewire; an optical fiber bundle disposed between the retractable sheath and the guidewire, the optical fiber bundle comprising a plurality of optical fibers adapted to transmit laser light energy in a laser ablation procedure. The optical fiber bundle comprises a first arm comprising a first plurality of optical fibers, and a second arm comprising a second plurality of optical fibers. The medical device further comprises an expansion device disposed on the guidewire and adapted to expand the optical fiber bundle from a first diameter to a second diameter.

In accordance with another representative embodiment, a medical device is disclosed. The medical device comprises a catheter comprising: a retractable sheath disposed over a guidewire; an optical fiber bundle disposed between the retractable sheath and the guidewire, the optical fiber bundle comprising a plurality of optical fibers adapted to transmit laser light energy in a laser ablation procedure. The optical fiber bundle comprises a first arm comprising a first plurality of optical fibers, and a second arm comprising a second plurality of optical fibers. A a density of optical fibers in at the first diameter is the same as a density of optical fibers at the second diameter. The medical device also comprises an expansion device disposed on the guidewire and adapted to expand the optical fiber bundle from a first diameter to a second diameter.

In accordance with another representative embodiments, a method of carrying out a medical procedure is disclosed. The method comprises: providing a catheter comprising: a retractable sheath disposed over a guidewire; and an optical fiber bundle comprising a plurality of optical fibers adapted to transmit laser light energy in a contacting medical procedure, wherein the optical fiber bundle comprises a first arm comprising a first plurality of optical fibers and a second arm comprising a second plurality of optical fibers; providing an expansion device adapted to expand optical fiber bundle from a first diameter to a second diameter; when the optical fiber has the first diameter, contacting plaque material on an interior wall of a vessel, and providing the laser light energy to the plaque material; and expanding the optical fiber bundle to the second diameter, contacting plaque material on the interior wall of the vessel, and providing the laser light energy to the plaque material.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1A is a perspective view of a medical device in a default position comprising catheter comprising an optical fiber bundle in accordance with a representative embodiment.
Fig. 1B is a perspective view of a medical device in an expanded position comprising catheter comprising an optical fiber bundle in accordance with a representative embodiment.
Fig. 2 is a perspective view of a medical device in accordance with a representative embodiment.
Figs. 3A-3E are perspective views showing deployment of the medical device and its application to remove plaque by contact in accordance with a representative embodiment.

### DETAILED DESCRIPTION

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises," and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" or "immediately adjacent" to another element or component, this encompasses only cases where the two elements or components are connected or disposed immediately adjacent to each other without any intermediate or intervening elements or components.

As described herein in connection with various representative embodiments, the present teachings relate generally to a medical device and its method of use for removing plaque from arteries. According to various representative embodiments described herein, the medical device comprises a laser catheter distal tip that is adapted to expand, illustratively diametrically to provide greater debulking in atherectomy procedures. As described more fully herein, optical fibers span the length of the medical device. At the proximal end of the medical device, the optical fibers are contained within the same outer jacket; at the distal end, the fibers are divided into a plurality of substantially identical inner jackets, which are terminated with the plurality of substantially identical distal bands (sometimes referred to herein as "arms") that interface to form a particularly shaped profile. In accordance with specific embodiments, there distal bands form a substantially circularly shaped profile. The retractable outer jacket provides a default (collapsed/unexpanded) profile in which the jacket is fully extended to contain the plurality of distal fiber bundles. In the default configuration, the distal end comprising the plurality of fiber bundles is comparatively small and is used to provide an initial ablation. As described more fully herein, the areal dimension (e.g., a circle) is comparatively small and is readily inserted into comparatively small access points (e.g., a radial artery). To expand the distal end of the medical device the outer jacket is retracted to enable expansion of the bundles.

As described more fully below, after deploying the catheter in the initial default configuration, the medical device is tracked to a target site, and functions as a conventional laser catheter of a constant (static) diameter. When the catheter reaches the treatment site in the default configuration, a pilot hole is first ablated still in the default configuration. After ablating the pilot hole, the outer sheath is retracted, and a secondary expansion accessory is deployed to separate the distal fiber bundles into an expanded state. At this point, the laser is then reactivated to contact a larger area of tissue and having the profile of the distal fiber bundles (arms) in the expanded state.

Among other clear improvements to the technical field of medical treatment, the medical devices and methods of the various representative embodiments include improvement of not only the area of plaque that can be removed in a procedure, but also the confidence of its complete removal. As alluded to above, the diameter of an atherectomy device comprising a laser energy source in known devices is largely constrained by the size of the anatomical access site. In the case of the radial approach for example, the typical introducer sheath is only 5-6F (approximately 1.9 - 2.2mm inner diameter), meaning that the catheter outer diameter should be even smaller to provide clearance. By extension, the potential luminal gain is constrained by the size of this access site as well, despite the vessel in need of treatment sometimes being 2 or 3 times larger than the access site (e.g., a typical femoropopliteal artery can be 5-6mm in diameter). As described more fully below, the medical devices of representative embodiments are first in a default configuration in which the diameter of the distal end of the catheter is suitable for anatomical site of initial deployment (e.g., a radial artery in an arm), but once deployed is expanded to contact and treat a larger area of plaque. As such, with its expanding distal end, the catheter can still access the treatment site via a variety of approaches while in the default (collapsed) state, yet the luminal gain is less limited by this constraint when compared to known medical devices.

Furthermore, as noted above, known laser catheters have a fixed outer diameter, limiting the area that can be ablated having a dimension (e.g., diameter) that is approximately the same size as the catheter for use in contact ablation in which only plaque that is physically touching the fiber optic face will interact with the laser light to be photoablated. By contrast, with the medical devices of the various representative embodiments, after ablating the plaque using the catheter in the default position, the expanded distal end of the catheter enables a greater area contacted and as a result ablation of a larger area of tissue creating a larger lumen. As described more fully below, the distal end of the expanded catheter is adapted to rotate to cover a larger area using the contact ablation. This provides clear improvements to the technical field of medical treatment

Further improvements to the technical field include clinician satisfaction. To this end, using known medical devices in current clinical cases, clinicians may often need to perform multiple passes with a laser catheter having a fixed distal end area in hopes of further debulking. Yet, even with multiple passes, the area ablated in contact ablation remains minimal. By contrast, the medical device comprising an expanding distal end of various representative embodiments, clinicians will have increased confidence that subsequent passes are contacting (and thereby ablating) more material with each pass. As will be appreciated by one of ordinary skill in the art, improving the area of debulking through application of the medical devices and method of various representative embodiments, benefits such as improved drug absorption into vessel walls due to increased contact area may result in less severe and fewer dissections may be realized, thereby reducing bailout stenting; and generally improving long term patency and reducing target lesion revascularization

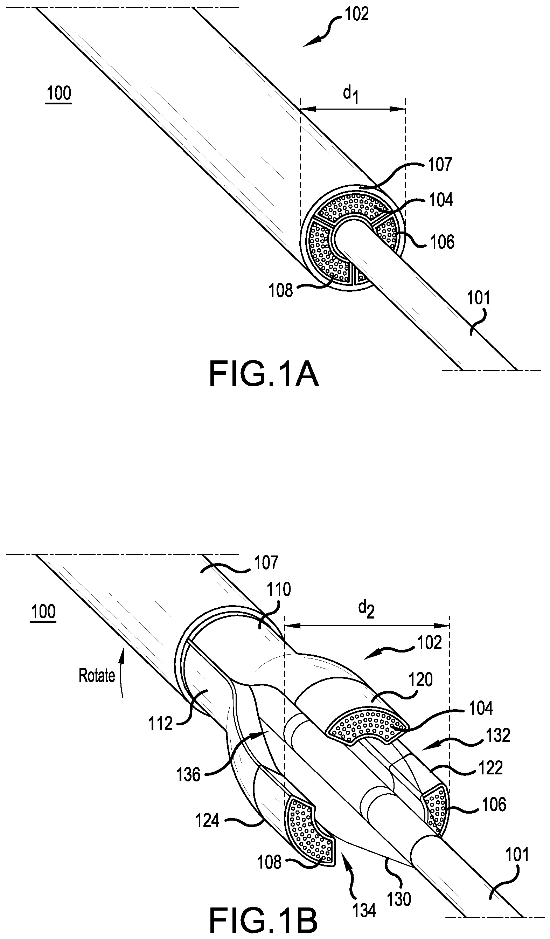

Fig. 1A is a perspective view of a medical device 100 in accordance with a representative embodiment. The medical device 100 comprises a catheter 102 with a guide wire 101 disposed therein. Optical fiber bundles 104, 106 and 108 are disposed in the catheter and around the guide wire 101 as shown. A retractable sheath 107 forms an outer layer of the catheter, and is retracted to provide the expanded configuration of the distal end of the medical device as described herein. The medical device 100 depicted in Fig. 1A is in the default configuration and illustratively has a circular profile having a diameter d₁. As noted above, in the default position, the diameter d₁ of the medical device is not only small enough to enable easy entry via a smaller access point (e.g., a radial artery), but also enables the first step of debulking by contact ablation of a pilot hole. Notably, in certain so-called standard laser catheters, d₁ is approximately 2.0 mm. This is merely illustrative, and d₁ may be greater or less than 2.0 mm. More generally, the diameter d₁ can be in the range of approximately 0.6 mm to approximately 3.4 mm.

Furthermore, while the general shape of the distal end of the catheter 102 is circular, this is merely illustrative because most catheters have a circular profile. Alternatively shaped profiles are contemplated by the present teachings. Generally, rounded profiles are contemplated. The present teaching also contemplate a clover or flower pattern distal end. Another shape contemplated would be an iris type design (e.g., like the iris of a camera lens), which would expand circularly but would beneficially reduce the gaps between arms in the expanded configuration (see the expanded configuration in Fig. 1B). In yet other contemplated embodiments, the expansion center could even be eccentric (i.e., the arms in Fig. 1B would be asymmetrical) to yield asymmetric expansion in the event that the plaque itself is eccentric within the vessel. The default configuration may be circular in this case, but the resulting expanded configuration would be asymmetric.

Notably, the number of optical fiber bundles shown in Fig. 1A is merely illustrative, and more or fewer optical fiber bundles than the three depicted are contemplated by the present teachings. In one illustrative embodiment, a single optical fiber bundle surrounding the guide wire 101 is contemplated. In this configuration, expansion of the distal end does not form so-called arms, but rather the distal end is expanded and expands the single optical fiber bundle to provide a circular profile having a greater diameter than d₁. Notably, other numbers of optical fiber bundles besides three or one are contemplated. Generally. there is a tradeoff in this design between the laser efficiency (both default and expanded configurations) versus manufacturability.

One possible expanded configuration includes each individual fiber free-floating and displaced by the expansion device, in a ring around the expansion device and with minimal gaps (or "dead space") between each fiber. While this may pose issues from the perspective of current manufacturing methods, such a configuration is contemplated. Rather, the current state of manufacturing involves epoxying fibers into a rigid distal band. There is therefore, a tradeoff of real-estate at the distal end: the surface area can be either fiber optics (which contribute to ablation), or the surface area of the distal bands themselves (more dead space). Furthermore, these band are already quite small and require significant skill to manufacture properly smaller, non-circular bands will only further complicate this process.

Notably, the catheter 102 could be either over the wire (OTW) or rapid exchange (RX). For OTW, the inner lumen may extend from a bifurcation at the proximal end of the working length until meeting the proximal junction of the fiber bundles, at which point the fiber bundles and distal bands themselves form the circular guidewire pathway. For an RX design, the proximal port may need to begin proximally of the fiber bundles, and also terminate at the internal proximal junction of the optical fiber bundles 104, 106, 108. Moreover, the catheter 102 could be made one of a number of lengths and/or diameter. The design of the optical fiber bundles 104, 106, 108 design is not impacted by the overall length of the catheter 102, and the design could be adapted for any overall diameter by simply increasing or decreasing the size of the optical fiber bundles 104, 106, 108 and distal bands (described below).

Once the distal end of the catheter 102 reaches a target, a laser (not shown) that is optically coupled to the optical fiber bundles 104, 106, 108 is activated, and ablation of a pilot hole having a diameter having a magnitude of approximately d₁ is made in the plaque or other tissue. Again, the ablation requires contact of the distal ends of the optical fiber bundles 104, 106, 108 with the tissue desired to be ablated.

Fig. 1B is a perspective view of the medical device 100 in an expanded configuration in accordance with a representative embodiment. Various aspects of the medical system and medical device described in connection with Fig. 1A are common to the presently described representative embodiments, and may not be repeated to avoid obscuring the presently described representative embodiments.

Turning to Fig. 1B, the retractable sheath 107 is shown in a retracted position, revealing an inner jacket 110 disposed on an outer portion of optical fiber bundle 104, and an inner jacket 112 disposed on the outer portion of the optical fiber bundle 108. Notably, the inner jacket disposed on an outer portion of optical fiber bundle 106 is not in view in Fig. 1B, but is substantially identical to inner jackets 110, 112.

A distal band 120 is disposed beginning near the distal end of optical fiber bundle 104. Similarly, a distal band 122 is disposed beginning near the distal end of optical fiber bundle 106; and distal band 124 is disposed beginning near the distal end of optical fiber bundle 108. The distal band illustratively comprises a metal, although other materials within the purview of the ordinarily skilled artisan are contemplated as well. In some embodiments the metal of distal bands 120, 122, 124 is ferromagnetic. As described more fully below, the use of ferromagnetic metal in distal band 120, 122, 124 is used to act with a magnet (not shown in Fig. 1B) to help in preventing splaying of the bundles when the catheter 102 is in the expanded configuration.

As shown, after retraction of the retractable sheath 107, an expansion device 130 is introduced by moving of the guide wire 101, as described more fully below. Generally, the expansion device 130 is deployable/retractable through the anatomy and capable of expanding the optical fiber bundles 104, 106, 108 as described more fully below. In accordance with a representative embodiment, the expansion device 130 may be a balloon, which is commonly used in procedures involving catheters deployed in vessels in the body (e.g., during an angioplasty procedure). The use of a balloon for the expansion device may be considered to be the easiest approach, considering that the crossing profile of the balloon is dynamic, i.e., the balloon can be deflated during delivery, inflated only during expansion of the optical fiber bundles 104, 106, 108, and deflated again for removal. The balloon could be a custom-made accessory to the expanding catheter, and may be available in different sizes depending on the use cases. In a representative embodiment, the balloon may be integrated into the catheter 102 itself, serving as an all-in-one solution. The balloon functions in the same way, except it would not be a separate accessory. However, this approach may present the challenge of limiting the possible area for optical fiber, and thereby ablation efficiency, to make room for the balloon instead. These factors have to be weighed in a cost-benefit manner when deciding whether to use this type of balloon.

Alternatively, the expansion device 130 may be a wedge made of a material compatible with use in living creatures. As described more fully below, the expansion device 130 illustratively comprises a sloped surface on each of its proximal and distal ends to prevent snagging of a vessel during deployment by the expansion device 130. Notably, having the slope surface on only the surface initially contacting the vessel is contemplated. In a representative embodiment, the expansion device is a substantially solid element made of a material suitable for applications of the medical device 100. One benefit of the wedge is a reduction in variation between procedures and possibly reduce procedure time by avoiding the inflation/deflation cycles. However, these benefits must be weighed against the fact that the clinician may have less control over the expansion process and a constant profile wedge may be more difficult to deliver to anatomy.

With the expansion device deployed, the three optical fiber bundles 104, 106, 108 disposed in the respective inner jackets and distal bands 120, 122, 124 form so-called "arms" of the catheter 102 that are separated by spaces 132, 134, 136 as shown. The separation of the arms of the catheter 102 results in a diameter d₂ of the distal end of the catheter 102. In this expanded configuration, the diameter d₂ of the distal end of the catheter 102 illustratively may be twice or thrice that of d₁ in the default configuration.

Once deployed in the expanded configuration, and the distal end of the catheter is disposed in a desired position of and in contact with the target, laser energy is provided to the optical fibers of each of the arms to ablate three sections of the target where the arms are in contact with the target.

Notably, and as can be readily appreciated from a reviews of Figs. 1A and 1B, the optical a density of optical fibers in each of the optical fiber bundles 104, 106, 108 at the first diameter is the substantially same as a density of optical fibers in each of the optical fiber bundles 104, 106, 108 at the second diameter. Stated somewhat differently, the density of the optical fibers in the optical fiber bundles 104, 106, 108 does not change because of the expansion realized by application of the expansion device 130. Accordingly, the laser energy power per unit area of contact with the tissue to be ablated is the substantially same in the expanded configuration of Fig. 1B as it is in the unexpanded configuration of Fig. 1A. Among other benefits, maintaining the same density of the optical fibers at the first diameter as at the second diameter results in substantially uniform ablation by the laser energy applied to the entire area ablated at both the first and second diameters.

After the ablation is carried out in the configuration of Fig. 1B, the catheter is rotated as through manual or automatic manipulation via a handle or similar known device (not shown) to locate the three arms where the spaces 132, 134, 136 exist in the configuration shown in Fig. 1B. In accordance with a representative embodiment, the medical device may be adapted to interface with an automated rotation handle, such that on the multiple passes of the expanded configuration, the fibers can contact new lesion material as described below. Alternatively, a manual torque attachment could enable rotations if desired.

Once deployed in the expanded configuration, and the distal end of the catheter is disposed in a desired position of and in contact with the target in spaces 132, 134, 136, laser energy is provided to the optical fibers of each of the arms to ablate these three sections of the target where the arms are in contact with the target. In this way, the ablated area approximating a circle having a diameter of approximately d₂ is realized. Accordingly, by initially ablating a pilot hole with the catheter in the default configuration of Fig. 1A, the expanded and rotated catheter provides a larger area of ablation of the tissue is carried out.

As alluded to above, the expansion of the distal end of the catheter 102 into three arms is merely illustrative. Rather more or fewer arms are contemplated by the representative embodiments of the present teachings and even an embodiment in which the optical fibers are a single component with no spaces is contemplated. As will be appreciated, the greater the number of optical fiber bundles, the smaller the area of ablation of each optical fiber bundle, and the lesser the number of optical fiber bundles, the greater the area of ablation of each optical fiber bundle. As will be appreciated, more of fewer rotations are made to ablate the entire diameter d₂; and in the case of a single optical fiber bundle comprising the entire shape (in this case a circle) that does not involve multiple optical fiber bundles, no rotations are needed.

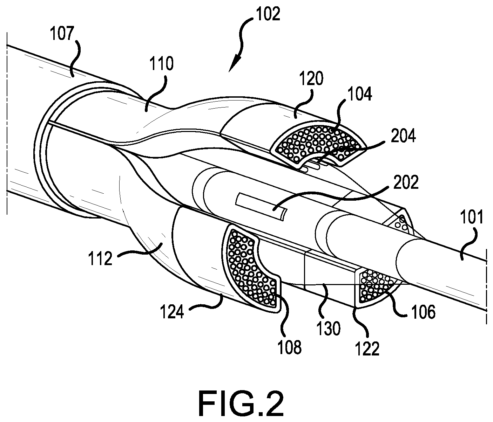

Fig. 2 is a perspective view of the medical device 100 in an expanded configuration in accordance with a representative embodiment. Various aspects of the medical system and medical device described in connection with Fig. 1B are common to the presently described representative embodiments, and may not be repeated to avoid obscuring the presently described representative embodiments.

Turning to Fig. 2, the retractable sheath 107 is shown in a retracted position, with the expansion device 130 positioned to expand the arms as described above. As alluded to above, splaying of the catheter 102 in the expanded position is beneficially avoided to the extent possible. Specifically, once the arms are in the expanded configuration as shown, it is beneficial for the arms of the medical device 100 be substantially prevented from "snagging" or dragging on the surrounding tissue as the catheter 102 is moved.

In one representative embodiment, the inner jackets (e.g., inner jackets 110, 112) material used for each optical fiber bundle 104, 106, 108 may be made of a semi-rigid or inherently "springy" material suitable for use in procedures involving the medical device 100. In this way, in the expanded configuration, the arms would bias towards their straight alignment, and would be naturally constrained toward the center of the circular cross section of the distal end of the catheter, remaining rather tight to the expansion tool.

In another representative embodiment, as alluded to above, the distal bands 120, 122, 124 could be made of a magnetic metal, and the expansion tool could contain a magnet. In the embodiment shown, a magnet 202 is provided in the guide wire 101. The magnetic attraction between the distal bands 120, 122, 124 and the magnet 202 keeps the optical fiber bundles 104, 106, 108 magnetically attracted / constrained to the expansion tool during treatment.

In yet another representative embodiment, splaying may be substantially prevented by deploying an elastic outer band (not shown) around the outer perimeter of the optical fiber bundles 104, 106, 108, thereby constraining the optical fiber bundles 104, 106, 108 concentrically when the catheter 102 is in the expanded configuration.

In yet another representative embodiment, grooves 204 may be provided on the fiber bundles to couple with the expansion device 130 such that the fiber bundles would slide along and be mechanically bound by the grooves, preventing the bundles from splaying outward.

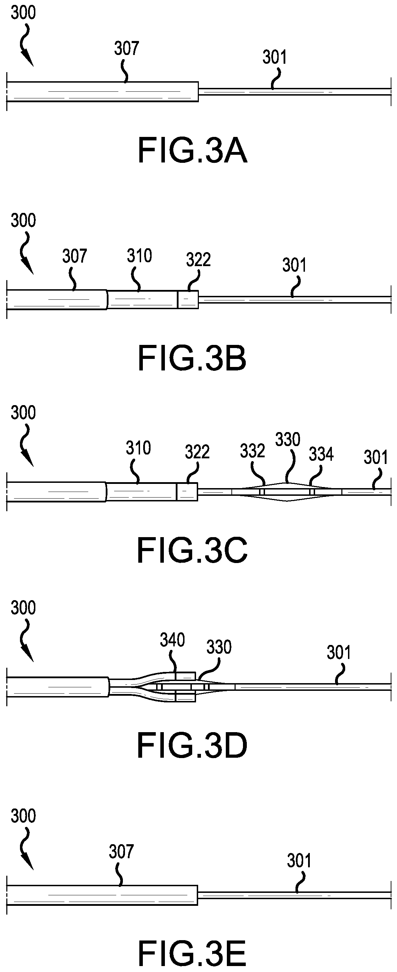

Figs. 3A-3E are perspective views showing deployment of the medical device and its application to remove plaque by contact in accordance with a representative embodiment. Various aspects of the medical system and medical device described in connection with Figs. 1A-2 are common to the presently described representative embodiments, and may not be repeated to avoid obscuring the presently described representative embodiments.

Turning to Fig. 3A, a medical device 300 is in the default configuration, with the retractable sheath 307 extended to the distal end of the optical fiber bundles (not shown in Fig. 3A). As noted above, once deployed, in the default configuration the distal ends of the optical fiber bundles are disposed in a way to be in contact with tissue to be ablated illustratively in the shape of a circle having the diameter d₁, and the pilot hole is formed.

In Fig. 3B, the retractable sheath 307 is retracted as described above, revealing an inner jacket 310 and a distal band 322. Notably, other distal bands are not shown in Fig. 3B.

Turning to Fig. 3C, an expansion device 330 is shown, having sloped sides 332, 334 on its proximal and distal ends (and above and below as shown). In a representative embodiment,

Fig. 3C shows a balloon used as the expansion device, with the balloon inflation occurring in Fig. 3C. In such a configuration, the guide wire 301 with the balloon may be deployed. By contrast, when a wedge is used for the expansion device 330, the guide wire 301 is first deployed, and the catheter 102 is deployed over the guide wire 301.

Turning to Fig. 3D, the medical device is shown in the expanded state. In this state, the arms 340 are disposed over the expansion device 330, and the ends of the optical fiber bundles are moved to contact with tissue to be ablated. As described above, after initial ablation, the catheter may be rotated to ablate a circle having a diameter d₂ as described above.

Turning to Fig. 3E, the medical device 300 is again in the default configuration after withdrawal of the expansion device and the return of the retractable sheath 307 to be flush with the distal end of the optical fiber bundles. In this configuration, the medical device 300 may be removed from the vessel.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

In summary, the application discloses a medical device that includes a catheter having a retractable sheath disposed over a guidewire. The catheter is adapted to transmit laser light energy in a medical procedure. The medical device further includes an expansion device adapted to expand an optical fiber bundle from a first diameter to a second diameter.

### Additional embodiments

Embodiment 1. A medical device, comprising a catheter, the catheter comprising: a retractable sheath disposed over a guidewire; an optical fiber bundle disposed between the retractable sheath and the guidewire, the optical fiber bundle comprising a plurality of optical fibers adapted to transmit laser light energy in a laser ablation procedure, wherein the optical fiber bundle comprises a first arm comprising a first plurality of optical fibers, and a second arm comprising a second plurality of optical fibers; and an expansion device disposed on the guidewire and adapted to expand the optical fiber bundle from a first diameter to a second diameter.
Embodiment 2. The medical device of embodiment 1, wherein the first arm comprises a first distal band, and the second arm comprises a second distal band adapted to prevent splaying of the first and second distal bands, respectively, during the expansion of the optical fiber bundle from the first diameter to the second diameter.
Embodiment 3. The medical device of embodiment 1, wherein at the second diameter, a gap exists between the first arm and the second arm.
Embodiment 4. The medical device of embodiment 1, wherein the optical fiber bundle further comprises a third arm comprising a third plurality of optical fibers.
Embodiment 5. The medical device of embodiment 1, wherein the optical fiber bundle is adapted to rotate to provide the laser light energy to an areal dimension having the second diameter.
Embodiment 6. The medical device of embodiment 1, wherein the expansion device comprises a balloon.
Embodiment 7. The medical device of embodiment 1, wherein the expansion device comprises a wedge.
Embodiment 8. The medical device of embodiment 7, wherein the wedge has sloping proximal and distal ends.
Embodiment 9. A medical device, comprising a catheter, the catheter comprising:
   a retractable sheath disposed over a guidewire; an optical fiber bundle disposed between the retractable sheath and the guidewire, the optical fiber bundle comprising a plurality of optical fibers adapted to transmit laser light energy in a laser ablation procedure, wherein the optical fiber bundle comprises a first arm comprising a first plurality of optical fibers, and a second arm comprising a second plurality of optical fibers, wherein a density of optical fibers at the first diameter is the same as a density of optical fibers at the second diameter; and an expansion device disposed on the guidewire and adapted to expand the optical fiber bundle from a first diameter to a second diameter.
Embodiment 10. The medical device of embodiment 9, wherein the first arm comprises a first distal band, and the second arm comprises a second distal band adapted to prevent splaying of the first and second distal bands, respectively, during the expansion of the optical fiber bundle from the first diameter to the second diameter.
Embodiment 11. The medical device of embodiment 9, wherein the optical fiber bundle is adapted to transmit the laser light energy at the first diameter and at the second diameter.
Embodiment 12. The medical device of embodiment 9, wherein at the second diameter, a gap exists between the first arm and the second arm.
Embodiment 13. The medical device of embodiment 9, wherein the optical fiber bundle further comprises a third arm comprising a third plurality of optical fibers.
Embodiment 14. The medical device of embodiment 9, wherein the optical fiber bundle is adapted to rotate to provide the laser light energy to an areal dimension having the second diameter.
Embodiment 15. The medical device of embodiment 9, wherein the expansion device comprises a balloon.

## Claims

1. A medical device, comprising:
a catheter comprising:
a retractable sheath disposed over a guidewire;
an optical fiber bundle disposed between the retractable sheath and the guidewire, the optical fiber bundle comprising a plurality of optical fibers adapted to transmit laser light energy in a laser ablation procedure, wherein the optical fiber bundle comprises a first arm comprising a first plurality of optical fibers, and a second arm comprising a second plurality of optical fibers; and
an expansion device disposed on the guidewire and adapted to expand the optical fiber bundle from a first diameter to a second diameter.

2. The medical device of claim 1, wherein the first arm comprises a first distal band, and the second arm comprises a second distal band adapted to prevent splaying of the first and second distal bands, respectively, during the expansion of the optical fiber bundle from the first diameter to the second diameter.

3. The medical device of claim 1 or 2, wherein at the second diameter, a gap exists between the first arm and the second arm.

4. The medical device of any one of claims 1 to 3, wherein the optical fiber bundle further comprises a third arm comprising a third plurality of optical fibers.

5. The medical device of any one of claims 1 to 4, wherein the optical fiber bundle is adapted to rotate to provide the laser light energy to an areal dimension having the second diameter.

6. The medical device of any one of the claims 1 to 5, wherein the expansion device comprises a balloon.

7. The medical device of any one of the claims 1 to 5, wherein the expansion device comprises a wedge.

8. The medical device of claim 7, wherein the wedge has sloping proximal and distal ends.

9. The medical device of any one of claims 1 to 8, wherein a density of optical fibers at the first diameter is the same as a density of optical fibers at the second diameter.

10. The medical device of any one of claims 1 to 9, wherein the optical fiber bundle is adapted to transmit the laser light energy at the first diameter and at the second diameter.

11. The medical device of any one of the claims 1 to 10 further comprising a handle configured to provide one or more of the following functions:
displace and/or rotate the retractable sheath relative to the guidewire; and
operated the expansion device to at least cause expansion of the optical fiber bundle.

12. A catheter as defined in any one of the claims 1 to 11.

13. The catheter of claim 12 configured to be comprised within a medical device.
